Europäisches Patentamt

(19) European Patent Office    (11) Numéro de publication: **0 195 870**

Office européen des brevets    **B1**

(12)    # FASCICULE DE BREVET EUROPEEN

(45) Date de publication du fascicule du brevet:
31.01.90

(21) Numéro de dépôt: **85400564.2**

(22) Date de dépôt: **22.03.85**

(51) Int. Cl.⁴: **A 61 K 35/72**

㊹    Utilisation de levures saccharomyces pour la fabrication

(43) Date de publication de la demande:
**01.10.86 Bulletin 86/40**

(45) Mention de la délivrance du brevet:
**31.01.90 Bulletin 90/5**

(84) Etats contractants désignés:
**AT BE CH DE FR GB IT LI LU NL SE**

(56) Documents cités:
**EP-A- 0 149 579**
**BE-A- 652 331**
**FR-A- 2 096 887**
**FR-A- 2 228 470**
**FR-M- 3 501**
**GB-A- 930 107**

**BIOLOGICAL ABSTRACTS/RMM, vol. 20, no. 15647, Philadelphia, PA, US; S. AHMAD et al.: "Induction of protective immunity against experimental infection of entamoeba-histolytica", & IRCS. (INT. RES. COMM. SYST.) MED. SCI.-LIB. COMPEND. 1980, vol. 9, no. 8, p526**
**BIOLOGICAL ABSTRACTS, vol. 79, 1985, no. 59018, Philadelphia, PA, US; A. SHARMA et al.: "Immunization of guinea pigs against entamoeba histolytica using glucan as an adjuvant", & INT. J. IMMUNOPHARMACOL 6(5): 483-492, 1984**

(73) Titulaire: **Laboratoires BIOCODEX, 19 rue Barbès, F-92126 Montrouge Cédex (FR)**

(72) Inventeur: **Gayral, Philippe, 52 avénue de la République, F-94800 Villejuif (FR)**
Inventeur: **Hublot, Bernard, 22 rue du Champ de Mars, F-75007 Paris (FR)**

(74) Mandataire: **Moncheny, Michel et al, c/o Cabinet Lavoix 2 Place d'Estienne d'Orves, F-75441 Paris Cedex 09 (FR)**

**Description**

La présente invention concerne un médicament contre l'amibiase à base de levures du genre Saccharomyces.

L'amibiase est une affection parasitaire et contagieuse due à un protozoaire, Entamoeba histolytica ou amibe dysentérique. On l'observe dans le monde entier, mais surtout dans les régions chaudes et humides, en particulier dans les pays tropicaux qui représentent le domaine électife de l'endémie amibienne. Cependant l'amibiase n'est pas rare même en climat tempéré, notamment à cause des grands brassages actuels de populations.

La forme la plus courante de cette affection est l'amibiase intestinale qui s'extériorise sous deux modes différents:
– la dysenterie amibienne, se manifestant par de violentes douleurs abdominales, des selles fréquentes faites de glaires et de sang, et ayant une tendance à la chronicité; et
– la colite amibienne chronique, plus habituelle en climat tempéré, se manifestant en particulier par une diarrhée chronique souvent alternée de constipation et de multiples troubles (dyspepsie gastrique ou hépato-biliaire, manifestations neuro-végétatives, etc. ...), et pouvant durer des années, même après disparition des amibes.

Les amibes peuvent aussi enrahir le foie, et plus rarement le poumon, et donc une forme grave de la maladie est l'hépatite amibienne.

Le traitement de l'amibiase se fait essentiellement par des amoebicides qui sont pour la plupart des dérivés de la quinoléine ou de l'arsenic qui sont mal tolérés. Les nitroimidazoles très efficaces sur les amibiases tissulaires ont une action insuffisante sur les formes intestinales. De plus, la prophylaxie de l'amibiase est difficile car le déparasitage des personnes de l'entourage atteintes est peu efficace. Il serait donc intéressante de disposer d'un agent amoebicide peu nocif et pouvant être utilisé facilement.

Par ailleurs, on sait que des levures du genre Saccharomyces, telles que Saccharomyces boulardii et Saccharomyces cerevisiae sont utilisée depuis longtemps dans la prévention et le traitement des troubles du tractus gastro-intestinal, en particulier la diarrhée ou colite associée à la prise d'antibiotiques ou à des séquelles d'amibiase (d. par exemple BE-A-652331 et FR-M-3501). Ces levures sont en général administrées par voie orale, sous forme de gélules contenant de 0,050 à 0,200 g de produit, les doses journalières étant habituellement entre 0,100 à 0,400g pour adulte.

On a maintenant trouvé que les levures du genre Saccharomyces, en particulier Saccharomyces Boulardii, réduisent de manière significative l'incidence et la gravité de l'amibiase chez les rongeurs, notamment chez le raton, dont l'amibiase caecale est reconnue comme modèle expérimental de celle du colon de l'homme (Woolfe G. Expérimental Chemotherapy vol. I, édité par R.L. Schinitzer et F. Hawking, 1963, Acad. Press., p. 422–443).

En conséquence, l'objet de l'invention est l'utilisation de levures revantes du genre Saccharomyces pour la fabrication d'un médicament contre l'amibiase aiguë. De préférence, les levures utilisées sont maintenues vivantes par lyophilisation. Elles peuvent être administrée notamment à une dose journalière de 0,5 à 10 g (basée sur leur poids à l'état lyophilisé). Plus spécialement, les levures utilisées selon l'invention appartiennent à l'espèce Saccharomyces boulardii.

Les levures peuvent être administrées par voie orale, à des doses unitaires allant de 0,250 à 2,5 g, par exemple sous forme de gélules contenant environ 0,250 g ou des sachets contenant de 0,250 à 2,5 g de levures.

Mais bien entendu d'autres formes de présentation, telles que les suspensions et d'autres voies d'administration, peuvent être utilisées.

Les exemples suivants illustrent l'invention, exemple pharmacologique

a) Matériel biologique

1. Amibes: Entamoeba histolytica, souche Rahman, en culture mixte avec des bactéries procaryotes. Les amibes sont cultivés à 37°C sur milieu diphasique (solution de Ringer enrichie en sérum de cheval recouvrant une pente de sérum coagulé et des grains d'amidon de riz) et sont associés avec des bactéries, en particulier des Citrobacter sp.

Le pouvoir pathogène est vérifié tous les mois par l'essai de l'abcès amibien hépatique du Hamster (voir Woolfe, ci-dessus). Les souches d'amibes sont systématiquement réisolées du foie et conservées en culture pendant quelques mois. En cas de baisse d'activité, une nouvelle souche est utilisée, isolée plus récemment d'un foie de Hamster.

2. Levures: Saccharomyces boulardii lyophilisées, provenance: Laboratoires Biocodex, (France). Les levures sont rehydratées dans du soluté de chlorure de sodium à 5% et activées par un choc thermique de 10 minutes à 25–30°C. Cette suspension contenant des levures vivantes est utilisée extemporanément.

Plusieurs préparations de Saccharomyces boulardii ont été comparées, levures vivantes, levures stérilisées par autoclavage et levures broyées, en choisissant une dose pour le traitement oral de 1,5 g/kg/jour.

3. Rats «C D» (Sprague-Dawley, Laboratoires Charles River France). Les animaux utilisés sont des ratons de 35 à 40 grammes, au sevrage, de l'un ou l'autre sexe. Ils sont nourris avec un lait en poudre courant, des pommes et ont de l'eau ad libitum. La veille de l'expérience, ils sont mis à la diète hydrique, après avoir reçu 0,1 ml/kg d'une solution saturée de sulfate de magnésium entraînant une certaine vacuité intestinale.

b) Inoculation des amibes:

Les ratons sont anesthésiés par injection intrapéritonéale de 25 mg/kg de pentobarbital sodique. Après laparotomie, la caecum est dégagé, légèrement irrité par frottement, et on y injecte environ

500 000 trophozoïtes d'Entamoeba histolytica sous un volume de 0,5 ml environ. La plaie chirurgicale est suturée en deux plans musculaire et cutané.

Les ratons récupèrent en 2 ou 3 heures et peuvent à nouveau être réunis dans ces cages d'élevage où ils continuent à recevoir lait en poudre, pommes et eau. Environ 10% des animaux meurent des suites de l'inoculation dans les premières 24 heures.

c) Traitement des ratons amibiens:

La levure est toujours administrée à la posologie de 1,5 g/kg/jour, en suspension dans une solutions de chlorure de sodium à 5 p 1 000 dans l'eau distillée; le traitement est effectué par canule gastrique en deux doses quotidiennes de 0,2 ml par raton à 10 heures et 18 heures. Les témoins ne reçoivent que la solution salée.

1. Essai curatif. Les ratons reçoivent les levures, des le lendemain de l'inoculation des amibes: ce traitement dure quatre jours (J2 à J5) mais il a pu être raccourci en fonction des résultats. Ces essais sont réalisés comparativement avec les levures vivantes, stérilisée ou broyées lors d'un traitement de quatre jours.

2. Essai préventif: La levure est administrée un à trois jours avant l'inoculation.

d) Appréciation quantitative du degré d'infection:

Les animaux sont sacrifiés le 6ème jour (J6) par anesthésie prolongée (éther) et autopsiés à l'aveugle. Le caecum est dégagé, fendu longitudinalement, et examiné.

La gravité de l'atteinte amibienne est appréciée par un indice entre 0 et 5 directement inspiré de celui de Woolfe mais en prenant plus en compte les facteurs de réaction tissulaire:

— l'aspect du caecum: normal ou rétracté, et l'épaisseur de la paroi normalement fine;

— le contenu de caecum: normalement consistant et granuleux, en particulier la recherche de mucus à l'interface paroi-contenu;

— la présence d'amibes: recherchées par examen microscopique direct du produit de raclage de la paroi caecale et dans le mucus et après culture.

On attribue à chaque animal, en fonction de son état pathologique un indice de gravité de 0 (indemnes d'amibes) à 5 (gravement, atteints).

Les résultats sont présentés par classes de gravité, ce qui permet de discuter de la répartition des rongeurs selon les traitements entre les différentes classes, par un test statistique du «chi-carré»:
— Indice 0: Animaux indemnes d'amibes (guéris ou non infectés)
— Indice 1-2-3: Animaux peu atteints.
— Indice 4-5: Animaux gravement atteints.

e) Résultats

Les résultats des différentes expérimentations sont groupés par essai dans le tableau suivant.

Tabelle

| Indices | Levures vivantes 1er essai | | Levures vivantes 2ème essai | |
|---|---|---|---|---|
| | Temoins | Traites | Temoins | Traites |
| 0 (Gueris) | 1 | 6 | 1 | 10 |
| 1-2-3 (Peu atteints) | 0 | 3 | 7 | 2 |
| 4-5 (Tres atteints) | 8 | 1 | 5 | 1 |
| Total des animaux | 9 | 10 | 13 | 13 |
| Signification Statistique | $X^2 = 12,01$ $p < 0,01$ | | $X^2 = 12,81$ $p < 0,01$ | |

Chez les ratons amibiens témoins ne recevant que l'excipient, on relève un taux faible de guérison de 10% environ. Les témoins ont toujours des indices plus élevés, 4 ou 5, car ils associent un caecum rétracté à paroi épaissie, la présence de mucus et celle d'Entamoeba histolytica confirmée par la culture.

Dans les essais d'activité curative, l'effet du traitement est net à deux niveau: augmentation du nombre d'animaux indemnes d'amibes et considérés comme guéris, et diminution du nombre d'animaux gravement atteints et d'indice élevé passant de 8 sur 9 chez les témoins à 1 sur 10 chez les traités.

Les études complémentaires ont montré que cet effet significatif de la levure se retrouve avec le produit autoclavé mais dans une moindre mesure; la levure broyée a une activité faible avec une diminution non significative du nombre de ratons très atteints; la durée de traitement nécessaire à l'activité curative pour une posologie de 1,5 g/kg/jour est de 3 jours. Dans les essais d'activité préventive, l'essai sur 2 ou 3 jours montre un effet modéré de la levure.

D'autre part, aucune toxicité imputable aux trois produits administrés n'a été relevée, soit par mortalité des rongeurs soit lors des traitements.

Chez le raton, les levures Saccharomyces boulardii vivantes possèdent un indéniable effet sur les populations de trophozoïdes d'Entamoeba histolytica et sur la pathologie caecale qu'ils induisent. La lésion de la paroi caecale étant reconnue proche de la lésion du colon de l'homme les résultats de cette expérimentation pharmacologique nous paraissent conforter à priori l'utilisation des levures de Saccharomyces contre l'amibiase chez l'homme.

Exemple de formation
Gélule contenant:
- cellules lyophylisées de
Saccharomyces boulardii       250 mg
- Stéarate de magnésium       3 mg

**Revendications**

1. Utilisation de levures vivantes du genre Saccharomyces pour la fabrication d'un médicament contre l'amibiase aiguë.

2. Utilisation selon la revendication 1, caractérisée en ce que les levures sont maintenues vivantes par lyophilisation.

3. Utilisation selon la revendication 1 ou la revendication 2, caractérisée en ce que les levures appartiennent à l'espèce Saccharomyces boulardii.

4. Utilisation selon l'une quelconque des revendications 1 à 3 en vue de l'administration par la voie orale, caractérisée en ce que des doses unitaires contenant de 0,250 à 2,5 g de levures sont préparées.

5. Utilisation selon la revendication 4, caractérisée en ce que la doses unitaires est sous forme de gélule contenant environ, 250 g de levures.

6. Utilisation selon la revendication 4, caractérisée en ce que la dose unitaire est sous forme de sachets contenant environ 0,250 à 2,5 g de levures.

**Patentansprüche**

1. Verwendung von lebenden Hefen des Genus Saccharomyces zur Herstellung eines Arzneimittels gegen die akute Amöbiase.

2. Verwendung nach Anspruch 1, dadurch gekennzeichnet, daß die Hefen durch Gefriertrocknen am Leben erhalten werden.

3. Verwendung nach Anspruch 1 oder 2, dadurch gekennzeichnet, daß die Hefen der Spezies Saccharomyces boulardii angehören.

4. Verwendung nach einem der Ansprüche 1 bis 3 zur Verabreichung auf oralem Wege, dadurch gekennzeichnet, daß Einheitsdosierungen, die 0,250 bis 2,5 g Hefen enthalten, hergestellt werden.

5. Verwendung nach Anspruch 4, dadurch gekennzeichnet, daß die Einheitsdosis in Form einer Gelkapsel vorliegt, die etwa 0,250 g der Hefen enthält.

6. Verwendung nach Anspruch 4, dadurch gekennzeichnet, daß die Einheisbasis in Form von Sachets vorliegt, die etwa 0,250 bis 2,5 g der Hefen enthalten.

**Claims**

1. Use of live yeasts of the genus Saccharomyces for the production of a drug to combat acute amoebiasis.

2. Use according to claim 1, characterised in that the yeasts are kept alive by lyophilisation.

3. Use according to claim 1 or 2, characterised in that the yeasts belong to the species Saccharomyces boulardii.

4. Use according to any one of claims 1 to 3 with a view to administration by oral route, characterised in that dosage units containing from 0.250 to 2.5 g of yeasts are prepared.

5. Use according to claim 4, characterised in that the dosage unit is in the form of a gelatin capsule containing about 0.250 g of yeast.

6. Use according to claim 4, characterised in that the dosage unit is in the form of sachets containing about 0.250 to 2.5 g of yeast.